(19) Europäisches Patentamt / European Patent Office / Office européen des brevets

(11) **EP 3 185 961 B1**

(12) **EUROPÄISCHE PATENTSCHRIFT**

(45) Veröffentlichungstag und Bekanntmachung des Hinweises auf die Patenterteilung:
**16.10.2019 Patentblatt 2019/42**

(21) Anmeldenummer: **15732247.0**

(22) Anmeldetag: **29.06.2015**

(51) Int Cl.:
*A61K 8/81* (2006.01)    *A61Q 5/06* (2006.01)

(86) Internationale Anmeldenummer:
**PCT/EP2015/064653**

(87) Internationale Veröffentlichungsnummer:
**WO 2016/030049 (03.03.2016 Gazette 2016/09)**

(54) **VERWENDUNG EINER KOMBINATION VON AQUASTYLE SH-100 UND LUVISKOL K90**

USE OF A COMBINATION OF AQUASTYLE SH-100 AND LUVISKOL K90

UTILISATION D'UNE ASSOCIATION D'AQUASTYLE SH-100 ET DE LUVISKOL K90

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **28.08.2014 DE 102014217206**

(43) Veröffentlichungstag der Anmeldung:
**05.07.2017 Patentblatt 2017/27**

(73) Patentinhaber: **Henkel AG & Co. KGaA
40589 Düsseldorf (DE)**

(72) Erfinder:
• **LANGE, Julia Bibiane
24641 Sievershütten (DE)**
• **RICHTERS, Bernd
21129 Hamburg (DE)**
• **BERMUDEZ AGUDELO, Maria Catalina
22089 Hamburg (DE)**
• **MARTINEZ, Cyrielle
22765 Hamburg (DE)**

(56) Entgegenhaltungen:
WO-A1-2015/095870    DE-A1- 19 947 684
DE-A1-102010 061 899    DE-A1-102012 223 978
US-A1- 2005 089 490    US-A1- 2007 202 068
US-A1- 2011 158 928    US-A1- 2014 093 467

• **Anonymous: "Ashland brings performance and style to crystal clear gel with AquaStyle(TM) (NYSE:ASH)", , 1. April 2014 (2014-04-01), XP055205095, Gefunden im Internet: URL:http://investor.ashland.com/releasedet ail.cfm?releaseid=836949 [gefunden am 2015-07-28]**
• **"Innovation Zone 2014", , 1. April 2014 (2014-04-01), Seiten 1-39, XP055205044, Gefunden im Internet: URL:http://www.in-cosmetics.com/RXUK/RXUK _ InCosmetics/2015-Website/Reviewpage_downlo ads/InnovationZoneGuide2014_lowres.pdf?v=6 35333310957041007 [gefunden am 2015-07-28]**

Bemerkungen:
Die Akte enthält technische Angaben, die nach dem Eingang der Anmeldung eingereicht wurden und die nicht in dieser Patentschrift enthalten sind.

**Beschreibung**

Die vorliegende Erfindung betrifft eine kosmetische Zusammensetzung zur Haarfestigung bzw. zur temporären Umformung von keratinischen Fasern, insbesondere menschlichen Haaren, wobei die Zusammensetzung eine Kombination eines anionischen Acrylatharzes und eines Vinylpyrrolidon-Homopolymers enthält.

Die temporäre Gestaltung von Frisuren für einen längeren Zeitraum bis hin zu mehreren Tagen erfordert in der Regel die Anwendung festigender Wirkstoffe. Daher spielen Haarbehandlungsmittel, die einer temporären Formgebung der Haare dienen, eine wichtige Rolle. Entsprechende Mittel zur temporären Verformung enthalten als festigenden Wirkstoff üblicherweise synthetische Polymere und/oder Wachse. Mittel zur Unterstützung der temporären Umformung keratinhaltiger Fasern können beispielsweise als Haarspray, Haarwachs, Haargel, Haarschaum konfektioniert werden.

Die wichtigste Eigenschaft eines Mittels zur temporären Verformung von Haaren, im Folgenden auch Stylingmittel genannt, besteht darin, den behandelten Fasern in der neu modellierten Form - d.h. einer den Haaren aufgeprägten Form - einen möglichst starken Halt zu geben. Man spricht auch von starkem Frisurenhalt oder vom hohen Haltegrad des Stylingmittels. Der Frisurenhalt wird im Wesentlichen durch die Art und Menge des eingesetzten festigenden Wirkstoffe bestimmt, wobei jedoch auch ein Einfluss der weiteren Bestandteile des Stylingmittels gegeben sein kann.

Neben einem hohen Haltegrad müssen Stylingmittel eine ganze Reihe weiterer Anforderungen erfüllen. Diese können grob in Eigenschaften am Haar, Eigenschaften der jeweiligen Formulierung, z.B. Eigenschaften des Schaums, des Gels oder des versprühten Aerosols, und Eigenschaften, die die Handhabung des Stylingmittels betreffen, unterteilt werden, wobei den Eigenschaften am Haar besondere Wichtigkeit zukommt. Zu nennen sind insbesondere Feuchtebeständigkeit, niedrige Klebrigkeit (tack) und ein ausgewogener Konditioniereffekt. Weiterhin soll ein Stylingmittel möglichst für alle Haartypen universell einsetzbar und mild zu Haar und Haut sein.

Um den unterschiedlichen Anforderungen gerecht zu werden, wurden als festigende Wirkstoffe bereits eine Vielzahl von synthetischen Polymeren entwickelt, die in Stylingmitteln zur Anwendung kommen. Die Polymere lassen sich in kationische, anionische, nichtionische und amphotere festigende Polymere unterteilen. Idealerweise ergeben die Polymere bei der Anwendung auf dem Haar einen Polymerfilm, der einerseits der Frisur einen starken Halt verleiht, andererseits aber hinreichend flexibel ist, um bei Beanspruchung nicht zu brechen. Ist der Polymerfilm zu brüchig, kommt es zur Bildung so genannter Filmplaken, das heißt Rückständen, die sich bei der Bewegung des Haares ablösen und den Eindruck vermitteln, der Anwender des entsprechenden Stylingmittels hätte Schuppen. Ähnliche Probleme ergeben sich, wenn Wachse als festigender Wirkstoff im Stylingmittel Einsatz finden. Handelt es sich bei dem Stylingmittel um ein Gel oder eine Paste, sollen die Polymere zudem verdickende Eigenschaften besitzen.

Bekannte anionische Polymere, die in Haarfestigungsprodukten Anwendung finden, sind hydrophob modifizierte Acrylatcopolymere. Diese sind im Handel erhältlich (INCI: Acrylates Copolymer (and) Water) und wirken im Wesentlichen als Verdickungsmittel. Das Datenblatt AquaStyle® SH-100 Polymer (Ashland Inc.) beschreibt ein solches Acrylatcopolymer und dessen Verwendung in Kombination mit Carbomeren. Es wird eine Eignung für kristallklare Haargele, eine gute Anfangssteifheit, Feuchtigkeitsbeständigkeit und eine Langzeitwirkung beschrieben.

Bekannte nichtionische Polymere, die in Haarfestigungsprodukten Anwendung finden, sind Polyvinylpyrrolidone (PVP). Es handelt sich dabei um Homopolymere von Vinylpyrrolidon. Vinylpyrrolidon-Homopolymere werden beispielsweise unter der Bezeichnung Luviskol® (BASF) vertrieben und werden üblicherweise als Filmbildner und/oder Verdickungsmittel eingesetzt. Insbesondere für die Verwendung in kosmetischen Zusammensetzungen und Stylingprodukten als Filmbildner oder Verdickungsmittel ist die Produktreihe Luviskol® K (BASF) erhältlich, in der Vinylpyrrolidon-Homopolymere in verschiedenen Molekulargewichten angeboten werden.

Aus der DE 10 2012 223978 A1 ist ein kosmetisches Mittel, a) mindestens ein Copolymer A aus den Monomeren a1) n-Butylmethacrylat a2) Methacrylsäure, a3) Ethylacrylat sowie gegebenenfalls weiteren Monomeren, b) mindestens ein Treibmittel und c) Wasser bekannt.

Die DE 19947684 A1 offenbart ein filmbildendes Hautreinigungsmittel, enthaltend einen teilactylierten Polyvinylalkohol mit 10-40 Mol% Vinylacetat-Gruppen als filmbildendes Polymer und ein Polyvinylpyrrolidon als adhäsives Polymer.

Die DE 10 2010 061 899 A1 offenbart Stylingmittel, die umfassend ein oder mehrere Treibgase (A), einen Alkohol (B), ein verdickendes Polymer (C) und ein haarfixierendes Polymer (D) umfasssen. Das haarfixierende Polymer ist gewählt aus Polyquatemium-89, Octylacrylamide/Acrylates/Butylaminoethylmethacrylate Copolymer, Acrylates/Hydroxyesters Acrylates Copolymer und/oder PVP und das verdickende Polymer ist Acrylic Acid/VP Crosspolymer.

Die US 2014/093467 beschreibt Mittel zur Haarbehandlung, enthaltend eine Kombination mindestens eines speziellen vernetzten amphiphilen, anionischen Polymers mit mindestens einem weiteren speziellen unvernetzten amphiphilen, anionischen Polymer und mindestens einem dritten Polymer. Ein ganz besonders bevorzugtes Polymer (a) ist ein vernetztes, amphiphiles, anionisches Polymer, das unter die INCI-Bezeichnung Acrylates / Steareth-20 Methacrylate Crosspolymer fällt. Das unvernetzte, anionische Polymer (b) ist vorzugsweise ein lineares Tetrapolymer aus Methacrylsäure, Hydroxyethylmethacrylat, Methylmethacrylat und Butylacrylat.

In der US 2005/089490 A wird ein Haarpflegemittel offenbart, welches (a) ein wasserlösliches oder wasserdispergierbares Styling-Polymer mit einer Glasübergangstemperatur unterhalb der Raumtemperatur, und (b) ein gelbildendes Polymer mit einer höher oder niedriger als Raumtemperatur, enthält.

In der Pressemitteilung "Ashland brings performance and style to crystal clear gel with AquaStyle" (http://investor.ashland.com/releasedetail.cfm?releaseid=836949) und der Messebroschüre zur incosmetics Messe 2014 (http://www.in-cosmetics.com/RXUK/RXUK_InCosmetics/2015-Website/Reviewpage_downloads/InnovationZoneGuide2014_lowres.pdf?v=635333310957041007) wird offenbart, dass der Einsatz des Stylingpolymers AquaStyle zu einem langanhaltender Frisurenhalt, einer hohen Feuchtigkeitsbeständigkeit der Frisur und einer einfachen Kämmbarkeit, ohne Schuppenbildung, führt.

Eine Aufgabe der vorliegenden Erfindung war es, weitere geeignete Polymerkombinationen zur Verfügung zu stellen, welche sich durch gute filmbildende und/oder festigende Eigenschaften auszeichnen, einen sehr hohen Haltegrad besitzen ohne dass dabei auf Flexibilität und gute Feuchtebeständigkeit - insbesondere Schweiß- und Wasserbeständigkeit - verzichtet werden müsste und sich zudem für die Herstellung stabil viskoser sowie stabil transparenter kosmetischer Zusammensetzungen eignen. Insbesondere sind derzeit erhältliche Stylingmittel noch dahingehend verbesserbar, dass eine gute Kombination aus Steifheit (Stiffness) und Langzeithalt nicht immer ausreichend gewährleistet wird. Es ist daher eine Aufgabe der vorliegenden Erfindung, derartige Stylingmittel bereitzustellen, die neben den oben genannten Eigenschaften insbesondere sowohl eine gute Steifheit als auch einen guten Langzeithalt ergeben.

Dies wurde erfindungsgemäß durch eine Kombination eines bestimmten anionischen Acrylatharzes und eines nichtionischen Polymers erreicht.

Durch die vorliegende Erfindung wird bereitgestellt:

1. Eine kosmetische Zusammensetzung zur temporären Umformung keratinischer Fasern, welche enthält:

(a) 0,01 bis 3,0 Gew.-% eines Vinylpyrrolidon-Homopolymers und
(b) 0,3 bis 2,0 Gew.-% mindestens eines anionischen Acrylatcopolymers (b), welches zumindest aus folgenden Monomereinheiten aufgebaut ist: mindestens einer (Meth)Acrylsäureeinheit (b1), mindestens einer (Meth)Acrylsäureethylestereinheit (b2) und mindestens einer (Meth)Acrylsäureestereinheit (b3), die von der (Meth)Acrylsäureethylestereinheit (b2) verschieden ist und eine hydrophobe Gruppe als Estergruppe aufweist und
(c) 0,02 bis 3 Gew.-% Homopolyacrylsäure.

2. Kosmetische Zusammensetzung nach Punkt 1, wobei das Vinylpyrrolidon-Homopolymer (a) einen K-Wert (1 Gew.-%ige Lösung von PVP, Brookfield bei 23[deg.]C) in Wasser von 20 bis 100 hat.

3. Kosmetische Zusammensetzung einem der vorhergehenden Punkte, wobei das Vinylpyrrolidon-Homopolymer (a) einen K-Wert von 80 bis 100, insbesondere etwa 90, hat.

4. Kosmetische Zusammensetzung nach einem der Punkte 1 bis 3, wobei das anionische Acrylatcopolymer (b) einen (Meth)Acrylsäurealkylester als Monomereinheit (b3) aufweist.

5. Kosmetische Zusammensetzung nach einem der vorhergehenden Punkte, wobei das anionische Acrylatcopolymer (b) Methacrylsäure als Monomereinheit (b1) und Ethylacrylat als Monomereinheit (b2) aufweist.

6. Kosmetische Zusammensetzung einem der vorhergehenden Punkte, wobei die Zusammensetzung weiterhin mindestens ein weiteres Verdickungsmittel enthält.

7. Kosmetische Zusammensetzung einem der vorhergehenden Punkte, wobei das anionische Acrylatcopolymer (b) bei einem Feststoffgehalt von 2 Gew.-% in einer wässerigen neutralisierten Lösung bei 25°C eine Viskosität von 60000 bis 120000 cPs aufweist.

8. Kosmetische Zusammensetzung einem der vorhergehenden Punkte, wobei das anionische Acrylatcopolymer (b) eines mit der INCI-Bezeichnung Acrylates Copolymer (and) Water, insbesondere AquaStyle™ SH-100 (Ashland Inc.), ist.

9. Kosmetische Zusammensetzung einem der vorhergehenden Punkte, wobei das Vinylpyrrolidon-Homopolymer (a) einen K-Wert von 80 bis 100, insbesondere etwa 90, hat und das anionische Acrylatcopolymer (b) eines mit der INCI-Bezeichnung Acrylates Copolymer (and) Water ist.

10. Kosmetische Zusammensetzung einem der vorhergehenden Punkte, wobei das anionische Acrylatcopolymer (b) AquaStyle™ SH-100 (Ashland Inc.) ist.

11. Kosmetische Zusammensetzung nach einem der vorhergehenden Punkte, enthaltend, bezogen auf das Gesamtgewicht der kosmetischen Zusammensetzung:

0,1 bis 2,0 Gew.-% des Vinylpyrrolidon-Homopolymers (a) und
0,8 bis 1,4 Gew.-% des anionischen Acrylatcopolymers (b).

12. Kosmetische Zusammensetzung nach einem der Punkte 6 bis 12, enthaltend, bezogen auf das Gesamtgewicht der kosmetischen Zusammensetzung:
0,05 bis 1,5 Gew.-% der Homopolyacrylsäure.

13. Kosmetische Zusammensetzung nach einem der vorhergehenden Punkte, wobei die Zusammensetzung als Haargel, Haarspray, Haarschaum oder Haarwachs, insbesondere als Haargel, vorliegt.

14. Verwendung einer kosmetischen Zusammensetzung nach einem der Punkte 1 bis 13 zur temporären Umformung keratinischer Fasern.

15. Verfahren zur temporären Verformung von keratinischen Fasern, insbesondere menschlichen Haaren, bei dem die kosmetische Zusammensetzung nach einem der Punkte 1 bis 13 auf keratinische Fasern appliziert wird.

Es wurde im Rahmen der vorliegenden Erfindung überraschend festgestellt, dass durch Kombination zweier an sich bekannter Polymere eine verbesserte Kombination aus Langzeitwirkung und Steifheit von Stylingprodukten, insbesondere von Haargelen, erhalten werden kann. Eine derartige Kombination von guter Steifheit und gutem Langzeithalt war nicht erwartbar.

Der Begriff keratinische Fasern umfasst erfindungsgemäß Pelze, Wolle und Federn, insbesondere aber menschliche Haare.

Die wesentlichen Bestandteile der erfindungsgemäßen kosmetischen Zusammensetzung sind das Vinylpyrrolidon-Homopolymer (a), das anionische Acrylatcopolymer (b) und eine Homopolyacrylsäure (c).

Das erfindungsgemäße Mittel enthält als Komponente (a) zwingend ein Vinylpyrrolidon-Homopolymer. Es ist erfindungsgemäß bevorzugt, die Vinylpyrrolidon-Homopolymere auszuwählen aus Vinylpyrrolidon- Homopolymeren mit einem K-Wert (1 Gew.-%igen Lösung von PVP, Brookfield bei 23[deg.]C) in Wasser von 20 bis 100. Bevorzugter ist ein K-Wert von 80 bis 100, bevorzugter etwa 90. Der auch als Eigenviskosität bezeichnete K-Wert ist ein aus der relativen Viskosität mittels Viskositätsmessungen von Polymerlösungen einfach zu bestimmender Parameter zur Charakterisierung von Polymeren.

Bevorzugte Vinylpyrrolidon-Homopolymere sind erhältlich unter dem Handelsnamen Luviskol® K 30, Luviskol® K 80, Luviskol® K 85, Luviskol® K 90, jeweils von der Firma BASF SE. Am bevorzugtesten ist erfindungsgemäß Luviskol® K 90. Luviskol® K90 ist eine 20 % wässerige, farblose bis leicht gelbliche Lösung von Polyvinylpyrrolidon. Das Produkt hat einen K-Wert von 90,0 bis 98,0 (1% (m/V) in Wasser), einen Feststoffgehalt von 19.0 bis 21.0 Gew.-% und einen pH-Wert von 7,0 bis 9,0 (10 Gew.-% Feststoffgehalt in Wasser).

Das anionische Acrylatcopolymer (b) ist zumindest aus folgenden Monomereinheiten aufgebaut: mindestens einer (Meth)Acrylsäureeinheit (b1), mindestens einer (Meth)Acrylsäureethylestereinheit (b2) und mindestens einer (Meth)Acrylsäureestereinheit (b3), die von der (Meth)Acrylsäureethylestereinheit (b2) verschieden ist und eine hydrophobe Gruppe als Estergruppe aufweist.

Das Copolymer (b) kann erfindungsgemäß aus weiteren Monomereinheiten aufgebaut sein. Gemäß Ausführungsformen der Erfindung ist das Copolymer (a) aber nur aus den Einheiten (b1), (b2) und (b3) aufgebaut, d. h. es besteht aus von diesen Monomereinheiten abgeleiteten Einheiten.

Die mindestens eine (Meth)Acrylsäureeinheit (b1) kann eine Methacrylsäure- oder Acrylsäureeinheit sein, wobei eine Methacrylsäureeinheit bevorzugt ist.

Die mindestens eine (Meth)Acrylsäureethylestereinheit (b2) kann eine Methacrylsäureethylestereinheit oder eine Acrylsäureethylestereinheit, wobei eine Acrylsäureethylestereinheit bevorzugt ist.

Die mindestens eine (Meth)Acrylsäureestereinheit (b3) kann erfindungsgemäß eine (Meth)Acrylsäurealkylestereinheit sein. Die Alkylgruppe der (Meth)Acrylsäurealkylestereinheit dient zur Steuerung der Hydrophobizität des Copolymers. Die Alkylgruppe ist bevorzugt eine lineare oder verzweigte Alkylgruppe mit 2 bis 30 Kohlenstoffatomen, bevorzugter 3 bis 12 Kohlenstoffatomen. Die hydrophobe Gruppe kann erfindungsgemäß auch eine andere hydrophobe als eine Alkylgruppe sein, z. B. eine aromatische Kohlenwasserstoffestergruppe. Als Beispiel sei eine substituierte oder unsubstituierte Phenylestergruppe oder substituierte oder unsubstituierte Alkylenphenylestergruppe genannt, z. B. eine Benzylestergruppe.

Die Viskosität des in der kosmetischen Zusammensetzung verwendeten anionischen Acrylatcopolymers (b) bei einem Feststoffgehalt von 2 Gew.-% und neutralisierter Lösung bei 25°C ist bevorzugt höchstens 60000 bis 120000 cPS.

Geeignete anionische Acrylatcopolymere (b) sind im Handel unter der INCI-Bezeichnung Acrylates Copolymer (and) Water erhältlich.

Am bevorzugtesten ist das anionische Acrylatcopolymer (b) AquaStyle® SH-100 Polymer von Ashland, Inc. Dieses hat in der im Handel erhältlichen Form einen Feststoffgehalt von etwa 28 bis 32 Gew.-% und einen pH-Wert von 2.1-4.0.

Die kosmetische Zusammensetzung der vorliegenden Erfindung enthält das Vinylpyrrolidon-Homopolymer (a) und Acrylatcopolymer (b) in für Stylingmittel üblichen und geeigneten Mengen, die für die spezielle Anwendung und Konfektionierung angepasst werden können.

Die erfindungsgemäße kosmetische Zusammensetzung enthält das Vinylpyrrolidon-Homopolymer (a), bezogen auf das Gesamtgewicht der kosmetischen Zusammensetzung, z. B. in einer Menge von 0,1 bis 3 Gew.-%, bevorzugt 0,1 bis 1,5 Gew.-%, weiterhin bevorzugt 0,2 bis 0,8 Gew.-%, jeweils angegeben als Feststoffgehalt aktiver Substanz in der kosmetischen Zusammensetzung.

Die erfindungsgemäße kosmetische Zusammensetzung enthält das Acrylatcopolymer (b), bezogen auf das Gesamtgewicht der kosmetischen Zusammensetzung, z. B. in einer Menge von 0,3 bis 2,0 Gew.-%, bevorzugt 0,8 bis 1,4 Gew.-%, jeweils angegeben als Feststoffgehalt aktiver Substanz in der kosmetischen Zusammensetzung.

In einer besonders bevorzugten Ausführungsform der vorliegenden Erfindung enthält die kosmetische Zusammensetzung als das Vinylpyrrolidon-Homopolymer (a) das im Handel unter der Bezeichnung Luviskol® K90 (BASF) erhältliche Copolymer und als das anionische Acrylatcopolymer (b) das im Handel unter der Bezeichnung AquaStyle™ SH-100 erhältliche Copolymer. Bei dieser Kombination wurden besonders gute Ergebnisse hinsichtlich einer Kombination von Steifheit und Langzeithalt erzielt. Besonders vorteilhaft ist diese Polymerkombination bei Stylingprodukten in Gelform. Weitere allgemein geforderte Eigenschaften von Stylingprodukten, wie z. B. Feuchtebeständigkeit und niedrige Klebrigkeit, werden insbesondere mit dieser Kombination gleichfalls erzielt, besonders bei Konfektionierung als Haargel.

Bevorzugt enthält die kosmetische Zusammensetzung der vorliegenden Erfindung eine oder mehrere weitere, als Verdickungsmittel oder Gelbildner wirkende Komponente(n), die von dem Vinylpyrrolidon-Homopolymer (a), dem Acrylatcopolymer (b) und der Homopolyacrylsäure (c) verschieden ist/sind und ebenfalls die Filmbildung unterstützen. Beispiele sind kationische, anionische, nichtionische oder amphotere Polymere. Der Gewichtsanteil dieser weiteren Komponente am Gesamtgewicht der kosmetischen Zusammensetzung kann aufgrund der Anwesenheit der Komponenten (a) und (b) vergleichsweise niedrig sein und beträgt beispielsweise 0,02 bis 3 Gew.-%, bevorzugt 0,05 bis 1,5 Gew.-% und noch bevorzugter 0,2 bis 0,8 Gew.-%.

Beispiele sind Acrylamide/Ammonium Acrylate Copolymer, Acrylamides/DMAPA Acrylates/Methoxy PEG Methacrylate Copolymer, Acrylamidopropyltrimonium Chloride/Acrylamide Copolymer, Acrylamidopropyltrimonium Chloride/Acrylates Copolymer, Acrylates/Acetoacetoxyethyl Methacrylate Copolymer, Acrylates/Acrylamide Copolymer, Acrylates/Ammonium Methacrylate Copolymer, Acrylates/t-Butylacrylamide Copolymer, Acrylates/C1-2 Succinates/Hydroxyacrylates Copolymer, Acrylates/Lauryl Acrylate/Stearyl Acrylate/Ethylamine Oxide Methacrylate Copolymer, Acrylates/Octylacrylamide Copolymer, Acrylates/Octylacrylamide/Diphenyl Amodimethicone Copolymer, Acrylates/Stearyl Acrylate/Ethylamine Oxide Methacrylate Copolymer, Acrylates/VA Copolymer, AcrylatesNP Copolymer, Adipic Acid/Diethylenetriamine Copolymer, Adipic Acid/Dimethylaminohydroxypropyl Diethylenetriamine Copolymer, Adipic Acid/Epoxypropyl Diethylenetriamine Copolymer, Adipic Acid/Isophthalic Acid/Neopentyl Glycol/Trimethylolpropane Copolymer, Allyl Stearate/VA Copolymer, Aminoethylacrylate Phosphate/Acrylates Copolymer, Aminoethylpropanediol-Acrylates/Acrylamide Copolymer, Aminoethylpropanediol-AMPD-Acrylates/Diacetoneacrylamide Copolymer, Ammonium VA/Acrylates Copolymer, AMPD-Acrylates/Diacetoneacrylamide Copolymer, AMP-Acrylates/Allyl Methacrylate Copolymer, AMP-Acrylates/C1-18 Alkyl Acrylates/C1-8 Alkyl Acrylamide Copolymer, AMP-Acrylates/Diacetoneacrylamide Copolymer, AMP-Acrylates/Dimethylaminoethylmethacrylate Copolymer, Bacillus/Rice Bran Extract/Soybean Extract Ferment Filtrate, Bis-Butyloxyamodimethicone/PEG-60 Copolymer, Butyl Acrylate/Ethylhexyl Methacrylate Copolymer, Butyl Acrylate/Hydroxypropyl Dimethicone Acrylate Copolymer, Butylated PVP, Butyl Ester of Ethylene/MA Copolymer, Butyl Ester of PVM/MA Copolymer, Calcium/Sodium PVM/MA Copolymer, Corn Starch/Acrylamide/ Sodium Acrylate Copolymer, Diethylene Glycolamine/Epichlorohydrin/Piperazine Copolymer, Dimethicone Crosspolymer, Diphenyl Amodimethicone, Ethyl Ester of PVM/MA Copolymer, Hydrolyzed Wheat Protein/PVP Crosspolymer, Isobutylene/Ethylmaleimide/Hydroxyethylmaleimide Copolymer, Isobutylene/MA Copolymer, Isobutylmethacrylate/Bis-Hydroxypropyl Dimethicone Acrylate Copolymer, Isopropyl Ester of PVM/MA Copolymer, Lauryl Acrylate Crosspolymer, Lauryl Methacrylate/Glycol Dimethacrylate Crosspolymer, MEA-Sulfite, Methacrylic Acid/Sodium Acrylamidomethyl Propane Sulfonate Copolymer, Methacryloyl Ethyl Betaine/Acrylates Copolymer, PEG/PPG-25/25 Dimethicone/Acrylates Copolymer, PEG-8/SMDI Copolymer, Polyacrylamide, Polyacrylate-6, Polybeta-Alanine/Glutaric Acid Crosspolymer, Polybutylene Terephthalate, Polyester-1, Polyethylacrylate, Polyethylene Terephthalate, Polymethacryloyl Ethyl Betaine, Polypentaerythrityl Terephthalate, Polyperfluoroperhydrophenanthrene, Polyquaternium-1, Polyquaternium-2, Polyquaternium-4, Polyquaternium-5, Polyquaternium-6, Polyquaternium-7, Polyquaternium-8, Polyquaternium-9, Polyquaternium-10, Polyquaternium-11, Polyquaternium-12, Polyquaternium-13, Polyquaternium-14, Polyquaternium-15, Polyquaternium-16, Polyquaternium-17, Polyquaternium-18, Polyquaternium-19, Polyquaternium-20, Polyquaternium-22, Polyquaternium-24, Polyquaternium-27, Polyquaternium-29, Polyquaternium-30, Polyquaternium-31, Polyquaternium-32, Polyquaternium-33, Polyquaternium-34, Polyquaternium-35, Polyquaternium-36, Polyquaternium-37, Polyquaternium-39, Polyquaternium-45, Polyquaternium-46, Polyquaternium-47, Polyquaternium-48, Polyquaternium-49, Polyquaternium-50, Polyquaternium-55, Polyquaternium-56, Polysilicone-9, Polyurethane-1, Polyurethane-6, Polyurethane-10, Polyvinyl Acetate, Polyvinyl Butyral, Polyvinylcaprolactam, Polyvinylformamide, Polyvinyl Imidazolinium Acetate, Polyvinyl Methyl Ether, Potassium Butyl Ester of PVM/MA Copolymer, Potassium Ethyl Ester of PVM/MA Copolymer, PPG-70 Polyglyceryl-10 Ether, PPG-12/SMDI Copolymer, PPG-51/SMDI Copolymer, PPG-10 Sorbitol, PVM/MA Copolymer, PVP, PVP/VA/Itaconic Acid Copolymer, PVP/VA/Vinyl Propionate Copolymer, Rhizobian Gum, Rosin Acrylate, Shellac, Sodium Butyl Ester of PVM/MA Copolymer, Sodium Ethyl Ester of PVM/MA Copolymer, Sodium Polyacrylate, Sterculia Urens Gum, Terephthalic Acid/Isophthalic Acid/Sodium Isophthalic Acid Sulfonate/Glycol Copolymer, Trimethylolpropane Triacrylate, Trimethylsiloxysilylcarbamoyl Pullulan, VA/Crotonates Copolymer, VA/Crotonates/Methacryloxybenzophenone-1 Copolymer, VA/Crotonates/Vinyl Neodecanoate Copolymer, VA/Crotonates/Vinyl Propionate Copolymer, VA/DBM Copolymer, VA/Vinyl Butyl Benzoate/Crotonates Copolymer, Vinylamine/Vinyl Alcohol Copolymer, Vinyl Caprolactam/VP/Dimethylaminoethyl Methacrylate Copolymer, VP/Acrylates/Lauryl Methacrylate Copolymer, VP/Dimethylaminoethylmethacrylate Copolymer, VP/DMAPA Acrylates Copolymer, VP/Hexadecene Copolymer, VP/VA Copolymer, VP/Vinyl Caprolactam/DMAPA Acrylates Copolymer, Yeast Palmitate und Styrene/VP Copolymer.

Beispiele für nichtionische Polymere sind:

- Vinylpyrrolidon/Vinylester-Copolymere, wie sie beispielsweise unter dem Warenzeichen Luviskol(BASF) vertrieben werden. LuviskolVA 64 und LuviskolVA 73, jeweils Vinylpyrrolidon/Vinylacetat-Copolymere, sind bevorzugte nichtionische Polymere.
- Celluloseether, wie Hydroxypropylcellulose, Hydroxyethylcellulose und Methylhydroxypropylcellulose, wie sie beispielsweise unter den Warenzeichen Culminalund Benecel (AQUALON) vertrieben werden.

- Schellack.
- Siloxane. Diese Siloxane können sowohl wasserlöslich als auch wasserunlöslich sein. Geeignet sind sowohl flüchtige als auch nichtflüchtige Siloxane, wobei als nichtflüchtige Siloxane solche Verbindungen verstanden werden, deren Siedepunkt bei Normaldruck oberhalb von 200 °C liegt. Bevorzugte Siloxane sind Polydialkylsiloxane, wie beispielsweise Polydimethylsiloxan, Polyalkylarylsiloxane, wie beispielsweise Polyphenylmethylsiloxan, ethoxylierte Polydialkylsiloxane sowie Polydialkylsiloxane, die Amin- und/oder Hydroxy-Gruppen enthalten.
- Glycosidisch substituierte Silicone.

Homopolyacrylsäure (INCI: Carbomer)ist im Handel unter dem Namen Carbopol® in unterschiedlichen Ausführungen erhältlich ist. Das Carbomer ist in einem Anteil von 0,02 bis 3 Gew.-%, bevorzugt 0,05 bis 1,5 Gew.-% und noch bevorzugter 0,2 bis 0,8 Gew.-%, in Bezug auf das Gesamtgewicht der kosmetischen Zusammensetzung, enthalten.

Die erfindungsgemäße kosmetische Zusammensetzung kann weitere übliche Stoffe von Stylingprodukten enthalten. Als weitere geeignete Hilfs- und Zusatzstoffe sind insbesondere zusätzliche Pflegestoffe zu nennen.

Als Pflegestoff kann das Mittel beispielsweise mindestens ein Proteinhydrolysat und/oder eines seiner Derivate enthalten. Proteinhydrolysate sind Produktgemische, die durch sauer, basisch oder enzymatisch katalysierten Abbau von Proteinen (Eiweißen) erhalten werden. Unter dem Begriff Proteinhydrolysate werden erfindungsgemäß auch Totalhydrolysate sowie einzelne Aminosäuren und deren Derivate sowie Gemische aus verschiedenen Aminosäuren verstanden. Das Molgewicht der erfindungsgemäß einsetzbaren Proteinhydrolysate liegt zwischen 75, dem Molgewicht für Glycin, und 200.000, bevorzugt beträgt das Molgewicht 75 bis 50.000 und ganz besonders bevorzugt 75 bis 20.000 Dalton.

Als Pflegestoff kann das erfindungsgemäße Mittel weiterhin mindestens ein Vitamin, ein Provitamin, eine Vitaminvorstufe und/oder eines derer Derivate enthalten. Dabei sind erfindungsgemäß solche Vitamine, Provitamine und Vitaminvorstufen bevorzugt, die üblicherweise den Gruppen A, B, C, E, F und H zugeordnet werden.

Wie auch der Zusatz von Glycerin und/oder Propylenglykol erhöht der Zusatz von Panthenol die Flexibilität des bei Anwendung des erfindungsgemäßen Mittels gebildeten Polymerfilms.

Als Pflegestoff können die erfindungsgemäßen Mittel weiterhin mindestens einen Pflanzenextrakt, aber auch Mono- bzw. Oligosaccharide und/oder Lipide enthalten.

Weiterhin sind als Pflegestoff Ölkörper geeignet. Zu den natürlichen und synthetischen kosmetischen Ölkörpern sind beispielsweise zu zählen pflanzliche Öle, flüssige Paraffinöle, Isoparaffinöle und synthetische Kohlenwasserstoffe sowie Di-n-alkylether mit insgesamt zwischen 12 bis 36 C-Atomen, insbesondere 12 bis 24 C-Atomen. Bevorzugte erfindungsgemäße kosmetische Mittel enthalten mindestens einen Ölkörper, vorzugsweise mindestens einen Ölkörper aus der Gruppe der Silikonöle. Zur Gruppe der Silikonöle zählen insbesondere die Dimethicone, zu welchen auch die Cyclomethicone zu rechnen sind, die aminofunktionellen Silikone sowie die Dimethiconole. Die Dimethicone können sowohl linear als auch verzweigt als auch cyclisch oder cyclisch und verzweigt sein. Geeignete Silikonöle oder Silikongums sind insbesondere Dialkyl- und Alkylarylsiloxane, wie beispielsweise Dimethylpolysiloxan und Methylphenylpolysiloxan, sowie deren alkoxylierte, quaternierte oder auch anionische Derivate. Bevorzugt sind cyclische und lineare Polydialkylsiloxane, deren alkoxylierte und/oder aminierte Derivate, Dihydroxypolydimethylsiloxane undPolyphenylalkylsiloxane.

Esteröle, das heißt Ester von C6-C30-Fettsäuren mit C2-C30-Fettalkoholen, vorzugsweise Monoester der Fettsäuren mit Alkoholen mit 2 bis 24 C-Atomen wie beispielsweise Isopropylmyristat (Rilanit® IPM), Isononansäure-C16-18-alkylester (Cetiol® SN), 2-Ethylhexylpalmitat (Cegesoft® 24), Stearinsäure-2-ethylhexylester (Cetiol® 868), Cetyloleat, Glycerintricaprylat, Kokosfettalkoholcaprinat/-caprylat (Cetiol® LC), n-Butylstearat, Oleylerucat (Cetiol® J 600), Isopropylpalmitat (Rilanit® IPP), Oleyl Oleate (Cetiol®), Laurinsäurehexylester (Cetiol® A), Di-n-butyladipat (Cetiol® B), Myristylmyristat (Cetiol® MM), Cetearyl Isononanoate (Cetiol® SN), Ölsäuredecylester (Cetiol® V) sind weitere bevorzugte pflegende Ölkörper.

Als Pflegestoffe eignen sich weiterhin Dicarbonsäureester, symmetrische, unsymmetrische oder cyclische Ester der Kohlensäure mit Fettalkoholen, Trifettsäureester von gesättigten und/oder ungesättigten linearen und/oder verzweigten Fettsäuren mit Glycerin oder Fettsäurepartialglyceride, worunter Monoglyceride, Diglyceride und deren technische Gemische zu verstehen sind.

Weiterhin sind in der erfindungsgemäßen Zusammensetzung bevorzugt Emulgatoren bzw. oberflächenaktive Mittel enthalten. Bevorzugt sind PEG-Derivate von hydriertem Ricinusöl, die z. B. unter der Bezeichnung PEG Hydrogenated Castor Oil erhältlich sind, z.B. PEG-30 Hydrogenated Castor Oil, PEG-33 Hydrogenated Castor Oil, PEG-35 Hydrogenated Castor Oil, PEG-36 Hydrogenated Castor Oil oder PEG-40 Hydrogenated Castor Oil. Erfindungsgemäß bevorzugt ist die Verwendung von PEG-40 Hydrogenated Castor Oil. Diese sind bevorzugt in einer Menge von 0,05 bis 1,5 Gew.-% enthalten, bevorzugter 0,1 bis 1,0 Gew.-%, ebenfalls bevorzugt 0,2 bis 0, 8 Gew.-% oder 0,3 bis 0,6 Gew.-%.

Die erfindungsgemäßen kosmetischen Mittel enthalten die Inhalts- bzw. Wirkstoffe in einem kosmetisch akzeptablen Träger.

Bevorzugte kosmetisch akzeptable Träger sind wässrige, alkoholische oder wässrig-alkoholische Medien mit vorzugsweise mindestens 10 Gew.-% Wasser, berechnet auf das Gesamtgewicht des Mittels.

Besonders bevorzugt enthält der erfindungsgemäße kosmetische Träger Wasser, insbesondere in der Menge, dass das kosmetische Mittel, berechnet auf das Gesamtgewicht des Mittels, mindestens 10 Gew.-%, insbesondere mindestens 20,0 Gew.-%, am bevorzugtesten mindestens 40 Gew.-% Wasser enthält.

Als Alkohole können insbesondere die für kosmetische Zwecke üblicherweise verwendeten niederen Alkohole mit 1 bis 4 Kohlenstoffatomen wie zum Beispiel Ethanol und Isopropanol enthalten sein.

Beispiele für wasserlösliche Lösungsmittel als Cosolvens sind Glycerin und/oder Ethylenglykol und/oder 1,2-Propylenglykol in einer Menge von 0 bis 30 Gew.-% bezogen auf das gesamte Mittel.

Die kosmetische Zusammensetzung der vorliegenden Erfindung kann in den für die temporäre Umformung von Haaren üblichen Formen konfektioniert sein, z. B. als Haargel, Haarspray Haarschaum oder Haarwachs. Bevorzugt ist die Konfektionierung als Haargel.

Sowohl Haarschäume als auch Haarsprays erfordern die Anwesenheit von Treibmitteln. Erfindungsgemäß sollten dafür jedoch bevorzugt keine oder nur geringe Mengen an Kohlenwasserstoffen eingesetzt werden. Dimethylether ist erfindungsgemäß ein geeignetes Treibmittel.

Die vorliegende Erfindung betrifft auch die Verwendung von erfindungsgemäßen kosmetischen Zusammensetzungen zur temporären Umformung von keratinischen Fasern, insbesondere von menschlichen Haaren, sowie ein Verfahren zur temporären Verformung von keratinischen Fasern, insbesondere menschlichen Haaren, bei dem die erfindungsgemäße kosmetische Zusammensetzung auf keratinische Fasern appliziert wird.

### Tabellarische Übersicht

| Die Zusammensetzung einiger bevorzugter kosmetischer Mittel kann den folgenden Tabellen entnommen werden (Angaben in Gew.-% bezogen auf das Gesamtgewicht des kosmetischen Mittels sofern nicht anders angegeben). | | | | | |
|---|---|---|---|---|---|
| | Formel 1* | Formel 2* | Formel 3* | Formel 4* | Formel 5* |
| Vinylpyrrolidon-Homopolymer (a) | 0,05 bis 3,0 | 0,1 bis 2,5 | 0,2 bis 2,0 | 0,3 bis 1,6 | 0,4 bis 1,4 |
| Copolymer (b) | 0,1 bis 5,0 | 0,2 bis 4,0 | 0,3 bis 2,0 | 0,6 bis 1,4 | 0,8 bis 1,2 |
| Misc | add 100 | add 100 | add 100 | add 100 | add 100 |
| | Formel 1a* | Formel 2a* | Formel 3a* | Formel 4a* | Formel 5a* |
| Vinylpyrrolidon-Homopolymer (a) mit K-Wert von 80 bis 100 (angegeben als Feststoffgehalt) | 0,05 bis 3,0 | 0,1 bis 2,5 | 0,2 bis 2,0 | 0,3 bis 1,6 | 0,4 bis 1,4 |

(fortgesetzt)

|  | Formel 1a* | Formel 2a* | Formel 3a* | Formel 4a* | Formel 5a* |
|---|---|---|---|---|---|
| Copolymer b): Acrylates Copolymer (and) Water | 0,1 bis 5,0 | 0,2 bis 4,0 | 0,3 bis 2,0 | 0,6 bis 1,4 | 0,8 bis 1,2 |
| Misc | add 100 | add 100 | add 100 | add 100 | add 100 |
|  | Formel 1b* | Formel 2b* | Formel 3b* | Formel 4b* | Formel 5b* |
| Vinylpyrrolidon-Homopolymer (a): Luviskol® K 90 - 20% (angegeben als Feststoffgehalt) | 0,05 bis 3,0 | 0,1 bis 2,5 | 0,2 bis 2,0 | 0,3 bis 1,6 | 0,4 bis 1,4 |
| Copolymer b): AquaStyle® SH-100 (angegeben als Feststoffgehalt) | 0,1 bis 5,0 | 0,2 bis 4,0 | 0,3 bis 2,0 | 0,6 bis 1,4 | 0,8 bis 1,2 |
| Misc | add 100 | add 100 | add 100 | add 100 | add 100 |
|  | Formel 11* | Formel 12* | Formel 13 | Formel 14 | Formel 15 |
| Vinylpyrrolidon-Homopolymer (a) | 0,05 bis 3,0 | 0,1 bis 2,5 | 0,2 bis 2,0 | 0,3 bis 1,6 | 0,4 bis 1,4 |
| Copolymer b) | 0,1 bis 5,0 | 0,2 bis 4,0 | 0,3 bis 2,0 | 0,6 bis 1,4 | 0,8 bis 1,2 |
| Carbomer | 0,02 bis 3,0 | 0,05 bis 2,0 | 0,05 bis 1,5 | 0,2 bis 1,0 | 0,35 bis 0,8 |
| Misc | add 100 | add 100 | add 100 | add 100 | add 100 |
|  | Formel 11a* | Formel 12a* | Formel 13a | Formel 14a | Formel 15a |
| Vinylpyrrolidon-Homopolymer (a) mit K-Wert von 80 bis 100 (angegeben als Feststoffgehalt) | 0,05 bis 3,0 | 0,1 bis 2,5 | 0,2 bis 2,0 | 0,3 bis 1,6 | 0,4 bis 1,4 |
| Copolymer b): Acrylates Copolymer (and) Water | 0,1 bis 5,0 | 0,2 bis 4,0 | 0,3 bis 2,0 | 0,6 bis 1,4 | 0,8 bis 1,2 |
| Carbomer | 0,02 bis 3,0 | 0,05 bis 2,0 | 0,05 bis 1,5 | 0,2 bis 1,0 | 0,35 bis 0,8 |
| Misc | add 100 | add 100 | add 100 | add 100 | add 100 |
|  | Formel 11b* | Formel 12b* | Formel 13b | Formel 14b | Formel 15b |
| Vinylpyrrolidon-Homopolymer (a): Luviskol® K 90 - 20% (angegeben als Feststoffgehalt) | 0,05 bis 3,0 | 0,1 bis 2,5 | 0,2 bis 2,0 | 0,3 bis 1,6 | 0,4 bis 1,4 |
| Copolymer b): AquaStyle® SH-100 (Angaben als Feststoffgehalt) | 0,1 bis 5,0 | 0,2 bis 4,0 | 0,3 bis 2,0 | 0,6 bis 1,4 | 0,8 bis 1,2 |
| Carbomer | 0,02 bis 3,0 | 0,05 bis 2,0 | 0,05 bis 1,5 | 0,2 bis 1,0 | 0,35 bis 0,8 |
| Misc | add 100 | add 100 | add 100 | add 100 | add 100 |
|  | Formel 21* | Formel 22* | Formel 23 | Formel 24 | Formel 25 |
| Vinylpyrrolidon-Homopolymer (a) | 0,05 bis 3,0 | 0,1 bis 2,5 | 0,2 bis 2,0 | 0,3 bis 1,6 | 0,4 bis 1,4 |
| Copolymer b) | 0,1 bis 5,0 | 0,2 bis 4,0 | 0,3 bis 2,0 | 0,6 bis 1,4 | 0,8 bis 1,2 |
| Carbomer | 0,02 bis 3,0 | 0,05 bis 2,0 | 0,05 bis 1,5 | 0,2 bis 1,0 | 0,35 bis 0,8 |
| PEG-40 Hydrogenated Castor Oil | 0,05 bis 1,5 | 0,1 bis 1,0 | 0,2 bis 0,9 | 0,3 bis 0,8 | 0,35 bis 0,6 |
| Misc | add 100 | add 100 | add 100 | add 100 | add 100 |
|  | Formel 21a* | Formel 22a* | Formel 23a | Formel 24a | Formel 25a |
| Vinylpyrrolidon-Homopolymer (a) mit K-Wert von 80 bis 100 (angegeben als Feststoffgehalt) | 0,05 bis 3,0 | 0,1 bis 2,5 | 0,2 bis 2,0 | 0,3 bis 1,6 | 0,4 bis 1,4 |

(fortgesetzt)

| | Formel 21a* | Formel 22a* | Formel 23a | Formel 24a | Formel 25a |
|---|---|---|---|---|---|
| Copolymer b): Acrylates Copolymer (and) Water | 0,1 bis 5,0 | 0,2 bis 4,0 | 0,3 bis 2,0 | 0,6 bis 1,4 | 0,8 bis 1,2 |
| Carbomer | 0,02 bis 3,0 | 0,05 bis 2,0 | 0,05 bis 1,5 | 0,2 bis 1,0 | 0,35 bis 0,8 |
| PEG-40 Hydrogenated Castor Oil | 0,05 bis 1,5 | 0,1 bis 1,0 | 0,2 bis 0,9 | 0,3 bis 0,8 | 0,35 bis 0,6 |
| Misc | add 100 | add 100 | add 100 | add 100 | add 100 |
| | Formel 21b* | Formel 22b* | Formel 23b | Formel 24b | Formel 25b |
| Vinylpyrrolidon-Homopolymer (a): Luviskol® K 90 - 20% (angegeben als Feststoffgehalt) | 0,05 bis 3,0 | 0,1 bis 2,5 | 0,2 bis 2,0 | 0,3 bis 1,6 | 0,4 bis 1,4 |
| Copolymer b): AquaStyle® SH-100 (Angaben als Feststoffgehalt) | 0,1 bis 5,0 | 0,2 bis 4,0 | 0,3 bis 2,0 | 0,6 bis 1,4 | 0,8 bis 1,2 |
| Carbomer | 0,02 bis 3,0 | 0,05 bis 2,0 | 0,05 bis 1,5 | 0,2 bis 1,0 | 0,35 bis 0,8 |
| PEG-40 Hydrogenated Castor Oil | 0,05 bis 1,5 | 0,1 bis 1,0 | 0,2 bis 0,9 | 0,3 bis 0,8 | 0,35 bis 0,6 |
| Misc | add 100 | add 100 | add 100 | add 100 | add 100 |
| * nicht erfindungsgemäß | | | | | |

Unter "Misc" ist erfindungsgemäß ein kosmetischer Träger zu verstehen, insbesondere Wasser und ggf. weitere übliche Bestandteile von Stylingprodukten.

Beispiele

1. Es wurde folgendes Stylinggel hergestellt:

| Zusammensetzung | 1 | 2* | | |
|---|---|---|---|---|
| Komponente/Rohstoff | Gew.-% | Gew.-% | INCI-Bezeichnung | Hersteller |
| Water, demineralized | 63,655 | 58,24 | | |
| Carbomer 20000-30000 mPas (0,2 %) | 0,39 | | Carbomer | |
| DMDM Hydantoin 55% | 0,1 | 0,1 | DMDM Hydantoin | |
| Sodium hydroxide pearls | 0,37 | 0,48 | | |
| Water, demineralized, ohne $H_2O_2$ | 29,705 | 29,7 | | |
| Luviskol® K90 - 20 % | 2 | 7 | Acrylates / Hydroxyesters Acrylates Copolymer | BASF |
| AquaStyle SH-100 | 3,3 | 3,3 | Acrylates Copolymer (and) Water | Ashland Inc. |
| Castor oil hydrog., 40 EO | 0,4 | 0,4 | PEG-40 Hydrogenated Castor Oil | |
| Parfum TEU-D-4106/6 Nutri Acai | 0,08 | 0,08 | | |
| Polygel HP / Synthalen HP | | 0,6 | Carbomer | 3V |
| Methylparaben | | 0,1 | | |
| **Gesamt** | 100 | 100 | | |
| * nicht erfindungsgemäß | | | | |

Die Mengenangaben in der Tabelle sind in Gew.-% des jeweiligen Rohstoffs, bezogen auf die gesamte Zusammensetzung, angegeben.

Das Haargel bildete bei Auftragen auf menschliches Haar einen transparenten Film mit sehr geringer Klebrigkeit.

Steifheit:

In eine trockene Haarsträhne (Euro-Naturhaar der Firma Kerling, 826500 Klebetresse dicht, einseitig geklebt, Gesamtlänge 150 mm, freie Länge 130 mm, Breite 20 mm, Gewicht 1,8 ± 0,2 g) wurden 850 mg einer gelförmigen Testzusammensetzung mit den Fingern einmassiert. Die mit der zu untersuchenden Testzusammensetzung behandelte Haarsträhne wird in einer Teflon-Schiene mit einem Durchmesser von 20 mm begradigt. Die präparierten Strähnen wurden anschließend über Nacht bei 21 [deg.]C und 50% relativer Luftfeuchte im Klimaraum getrocknet und konditioniert.

Die konditionierte Strähne wurde vorsichtig aus der Teflon-Schiene genommen. Die entstandene flache Strähne wurde auf die 40mm voneinander entfernten Messblöcke gelegt. Darüber wird mittig der 3PB Adapter eines Universalprüfgeräts AMETEK LF Plus der Firma AMETEK Precision Instruments Europe GmbH, Produktgruppe Lloyd montiert. Die gesamte Messung erfolgte im Klimaraum unter konstanten klimatischen Bedingungen bei 21 [deg.]C und 50% relativer Luftfeuchte.

Um standardisierte Ausgangsbedingungen zu schaffen, startete die Messung mit dem Anfahren einer Vorlast von 0,05 N. Anschließend wurde die Strähne mit einer Geschwindigkeit von 500 mm min$^{-1}$ um 15 mm gedrückt, wobei die dazu nötige Kraft gemessen wird. Nachdem die charakteristische Kraft K bei der maximalen Deformation von 15 mm aufgezeichnet wurde.

Mit dieser Messmethode lässt sich anhand der Kraft Fmax als Parameter der Steifheit des Polymerfilms und der Haltegrad des dadurch generierten Frisurenhalts messen.

Pro Testzusammensetzungen wurden 10 Strähnen erstellt und vermessen. Es wurden Steifheitswerte im Bereich von 2,3 -3,8 N erhalten, wobei es sich um arithmetische Mittel handelte.

Langzeithalt:

Die Zusammensetzung wurde mittels einer Long Lasting Hold Messung hinsichtlich ihrer formgebenden Eigenschaften überprüft. Hierzu wurden standardisierte Haarsträhnen der Fa. Kerling (Art. Nr. 826500) des Haartyps "European Natural, Farbe 6/0) von einer Länge ($L_{max}$) von 220 mm und einem Gewicht von 3,0 g eingesetzt. Zur Vorbereitung wurden die Strähnen mit einer 12,5 Gew.-%igen Natriumlaurethsulfat-Lösung gewaschen. Die Haarsträhnen wurden über Nacht in einem Trockenofen bei 318 K getrocknet.

Die Haare wurden 20 min in lauwarmen Wasser eingeweicht und dann auf ca. 50% Restfeuchte im Haare abgetupft,

750 mg der Zusammensetzung wurden auf jede der Haarsträhnen aufgebracht und einmassiert. Die Haarsträhnen wurden in eine Teflonschiene eingelegt, mittels einer Stahlrolle geglättet und über Nacht bei 21 [deg.]C und 50% getrocknet.

Die Haarsträhnen wurden nachfolgend an ihrem eine Ende in eine Haltevorrichtung eingespannt, und für eine Dauer von sechs Stunden bei 21 [deg.]C und 85% relativer Luftfeuchte gelagert. Zur Berechnung des Long Lasting Hold (LLH) wurden die aus der Haltevorrichtung herausragende Strähnenlänge vor ($L_0$) und nach ($L_t$) der Lagerung gemessen.

Der Long Lasting Hold ist ein Maß für die zeitliche Längenänderung einer mittels eines Haarverformungsmittels fixierten Haarsträhne. Je höher der LLH-Wert, desto geringer die Längenänderung der Haarsträhne unter Einfluss von Luftfeuchtigkeit in einer bestimmten Zeitdauer und desto besser der Haltegrad des Haarverformungsmittels.

Der Long Lasting Hold wurde nach folgender Formel berechnet.

$$LLH = 1 - (L_t - L_0 / L_{max})$$

Es wurde ein LLH-Werte von 40-60% ermittelt (arithmetisches Mittel der LLH-Werte von zehn Teststrähnen).

Das erfindungsgemäße Gel zeigte somit eine außergewöhnlich gute Kombination aus Langzeithalt und Steifheit.

**Patentansprüche**

**1.** Kosmetische Zusammensetzung zur temporären Umformung keratinischer Fasern, welche enthält:

    (a) 0,01 bis 3,0 Gew.-%_mindestens eines Vinylpyrrolidon-Homopolymers,
    (b) 0,3 bis 2,0 Gew.-%_mindestens eines anionischen Acrylatcopolymers (b), welches zumindest aus folgenden Monomereinheiten aufgebaut ist: mindestens einer (Meth)Acrylsäureeinheit (b1), mindestens einer (Meth)Acrylsäureethylestereinheit (b2) und mindestens einer (Meth)Acrylsäureestereinheit (b3), die von der (Meth)Acrylsäureethylestereinheit (b2) verschieden ist und eine hydrophobe Gruppe als Estergruppe aufweist, und (c) 0,02 bis 3 Gew.-% Homopolyacrylsäure.

2. Kosmetische Zusammensetzung nach Anspruch 1, wobei das Vinylpyrrolidon-Homopolymer (a) einen K-Wert (1 Gew.-%ige Lösung von PVP, Brookfield bei 23[deg.]C) in Wasser von 20 bis 100 hat.

3. Kosmetische Zusammensetzung Anspruch 1 oder 2, wobei das Vinylpyrrolidon-Homopolymer (a) einen K-Wert von 80 bis 100, insbesondere etwa 90, hat.

4. Kosmetische Zusammensetzung nach einem der vorhergehenden Ansprüche, wobei das anionische Acrylatcopolymer (b) einen (Meth)Acrylsäurealkylester als Monomereinheit (b3) aufweist.

5. Kosmetische Zusammensetzung einem der vorhergehenden Ansprüche, wobei das Vinylpyrrolidon-Homopolymer (a) einen K-Wert von 80 bis 100, insbesondere etwa 90, hat und das anionische Acrylatcopolymer (b) eines mit der INCI-Bezeichnung Acrylates Copolymer (and) Water, insbesondere AquaStyle™ SH-100 (Asghland, Inc.), ist.

6. Kosmetische Zusammensetzung nach einem der vorhergehenden Ansprüche, enthaltend, bezogen auf das Gesamtgewicht der kosmetischen Zusammensetzung:

   0,1 bis 2,0 Gew.-% des Vinylpyrrolidon-Homopolymers (a) und
   0,8 bis 1,4 Gew.-% des anionischen Acrylatcopolymers (b).

7. Verwendung einer kosmetischen Zusammensetzung nach einem der Ansprüche 1 bis 6 zur temporären Umformung keratinischer Fasern.

8. Verfahren zur temporären Verformung von keratinischen Fasern, insbesondere menschlichen Haaren, bei dem die kosmetische Zusammensetzung nach einem der Ansprüche 1 bis 6 auf keratinische Fasern appliziert wird.


**Claims**

1. A cosmetic composition for temporarily shaping keratin fibers, containing:

   (a) 0.01 to 3.0 wt.% of at least one vinylpyrrolidone homopolymer,
   (b) 0.3 to 2.0 wt.% of at least one anionic acrylate copolymer (b) which is made up of at least the following monomer units: at least one (meth)acrylic acid unit (b1), at least one (meth)acrylic acid ethyl ester unit (b2), and at least one (meth)acrylic acid ester unit (b3) which is different from the (meth)acrylic acid ethyl ester unit (b2) and comprises a hydrophobic group as the ester group, and
   (c) 0.02 to 3 wt.% homopolyacrylic acid.

2. The cosmetic composition according to claim 1, wherein the vinylpyrrolidone homopolymer (a) has a K value (1 wt.% solution of PVP, Brookfield at 23 °C) in water of 20 to 100.

3. The cosmetic composition according to claim 1 or 2, wherein the vinylpyrrolidone homopolymer (a) has a K value of 80 to 100, in particular approximately 90.

4. The cosmetic composition according to one of the preceding claims, wherein the anionic acrylate copolymer (b) comprises a (meth)acrylic acid alkyl ester as the monomer unit (b3).

5. The cosmetic composition according to one of the preceding claims, wherein the vinylpyrrolidone homopolymer (a) has a K value of 80 to 100, in particular approximately 90, and the anionic acrylate copolymer (b) is a copolymer that has the INCI name Acrylates Copolymer (and) Water, in particular AquaStyle™ SH-100 (Asghland, Inc.).

6. The cosmetic composition according to one of the preceding claims, containing, based on the total weight of the cosmetic composition:

0.1 to 2.0 wt.% of the vinylpyrrolidone homopolymer (a) and
0.8 to 1.4 wt.% of the anionic acrylate copolymer (b).

7. The use of a cosmetic composition according to one of claims 1 to 6 for temporarily shaping keratin fibers.

8. A method for temporarily shaping keratin fibers, in particular human hair, in which the cosmetic composition according to one of claims 1 to 6 is applied to keratin fibers.


**Revendications**

1. Composition cosmétique destinée à transformation temporaire des fibres kératiniques, comprenant :

   (a) 0,01 à 3,0 % en poids d'au moins un homopolymère de vinylpyrrolidone,
   (b) 0,3 à 2,0 % en poids d'au moins un copolymère d'acrylate anionique (b) qui est composé au moins des unités de monomères suivantes : au moins une unité d'acide (méth)acrylique (b1), au moins une unité d'ester éthylique d'acide (méth)acrylique (b2) et au moins une unité d'ester d'acide (méth)acrylique (b3) différente de l'unité d'ester éthylique d'acide (méth)acrylique (b2) et comportant un groupe hydrophobe comme groupe ester, et
   (c) 0,02 à 3 % en poids d'acide homopolyacrylique.

2. Composition cosmétique selon la revendication 1, dans laquelle l'homopolymère de vinylpyrrolidone (a) a une valeur K (solution à 1 % en poids de PVP, Brookfield à 23[deg.]C) dans de l'eau de 20 à 100.

3. Composition cosmétique selon la revendication 1 ou 2, dans laquelle l'homopolymère de vinylpyrrolidone (a) a une valeur K de 80 à 100, en particulier d'environ 90.

4. Composition cosmétique selon l'une des revendications précédentes, dans laquelle le copolymère d'acrylate anionique (b) comporte un ester alkylique d'acide (méth)acrylique comme unité monomère (b3).

5. Composition cosmétique selon l'une des revendications précédentes, dans laquelle l'homopolymère de vinylpyrrolidone (a) a une valeur K de 80 à 100, en particulier d'environ 90, et le copolymère d'acrylate anionique (b) est un copolymère portant la nomenclature INCI Acrylates Copolymer (and) Water, en particulier AquaStyle™ SH-100 (Ashland Inc.).

6. Composition cosmétique selon l'une des revendications précédentes, contenant, par rapport au poids total de la composition cosmétique :

   0,1 à 2,0 % en poids de l'homopolymère de vinylpyrrolidone (a) et
   0,8 à 1,4 % en poids du copolymère d'acrylate anionique (b).

7. Utilisation d'une composition cosmétique selon l'une des revendications 1 à 6 pour la mise en forme temporaire de fibres kératiniques.

8. Procédé de mise en forme temporaire de fibres kératiniques, en particulier de cheveux humains, dans lequel la composition cosmétique selon l'une des revendications 1 à 6 est appliquée sur des fibres kératiniques.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102012223978 A1
- DE 19947684 A1
- DE 102010061899 A1
- US 2014093467 A
- US 2005089490 A

**In der Beschreibung aufgeführte Nicht-Patentliteratur**

- *Ashland brings performance and style to crystal clear gel with AquaStyle, http://investor.ashland.com/releasedetail.cfm?releaseid=836949*
- *Messebroschüre zur incosmetics Messe,* 2014, http://www.in-cosmetics.com/RXUK/RXUK_InCosmetics/2015-Website/Reviewpage_downloads/InnovationZoneGuide2014_lowres.pdf?v=635333310957041007